(19) Europäisches Patentamt
European Patent Office
Office européen des brevets

(11) Veröffentlichungsnummer: **0 202 527**
**B1**

(12) # EUROPÄISCHE PATENTSCHRIFT

(45) Veröffentlichungstag der Patentschrift: **27.09.89**

(51) Int. Cl.[4]: **C 07 D 401/04**, C 07 D 401/14, C 07 H 15/26, A 61 K 31/44, A 61 K 31/70

(21) Anmeldenummer: **86106118.2**

(22) Anmeldetag: **05.05.86**

(54) **Pyrimidinyl-dihydropyridine, Verfahren zur Herstellung und ihre Verwendung in Arzneimitteln.**

(30) Priorität: **15.05.85 DE 3517473**

(43) Veröffentlichungstag der Anmeldung: **26.11.86 Patentblatt 86/48**

(45) Bekanntmachung des Hinweises auf die Patenterteilung: **27.09.89 Patentblatt 89/39**

(84) Benannte Vertragsstaaten: **AT BE CH DE FR GB IT LI NL SE**

(56) Entgegenhaltungen: **EP-A- 0 186 028**

(73) Patentinhaber: **BAYER AG, D-5090 Leverkusen 1 Bayerwerk (DE)**

(72) Erfinder: **Franckowiak, Gerhard, Dr., Henselweg 10, D-5600 Wuppertal 1 (DE)**
Erfinder: **Bechem, Martin, Dr., Obere Bergerheide 4, D-5600 Wuppertal 1 (DE)**
Erfinder: **Gross, Rainer, Dr., Platzhoffstrasse 23, D-5600 Wuppertal 1 (DE)**
Erfinder: **Kayser, Michael, Dr., Fleyerstrasse 231, D-5800 Hagen 1 (DE)**
Erfinder: **Schramm, Matthias, Dr., Paffrather Strasse 38, D-5000 Köln 80 (DE)**
Erfinder: **Thomas, Günter, Dr. c/o Bayer Italia S.p.A., Rep. Pharmacologia Via delle Groane 126, I-20024 Garbagnate Milano (IT)**

**EP 0 202 527 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft 4-Pyrimidinyl-1,4-dihydropyridine, Verfahren zu ihrer Herstellung sowie ihre Verwendung in Arzneimitteln, insbesondere in kreislaufbeeinflussenden Arzneimitteln.

Es ist bekannt, daß 1,4-Dihydropyridine als Calciumantagonisten eine Hemmung der Kontraktionskraft von glatten und kardialen Muskeln bewirken und zur Behandlung von Coronar- und Gefäßerkrankungen eingesetzt werden können [vgl. A. Fleckenstein, Am. Rec. Pharmacol. Toxicol. 17, 149–166 (1977)].

In der Europäischen Patentanmeldung 186028, die am 2.7.1986 publiziert wurde, werden bereits ähnliche 5-Nitrodihydropyridine mit kreislaufbeeinflussender Wirkung beschrieben. Die vom Anspruch der vorliegenden Erfindung durch Disclaimer ausgenommen werden.

Bei Kenntnis des vorpublizierten Standes der Technik war nicht zu erwarten, daß die erfindungsgemäßen Verbindungen aus dieser Stoffklasse keine kontraktionshemmende, sondern eine kontraktionskraftverstärkende, am Herzmuskel positiv inotrope Wirkung besitzen.

Die vorliegende Erfindung betrifft 4-Pyrimidinyl-1,4-dihydropyridine der allgemeinen Formel (I)

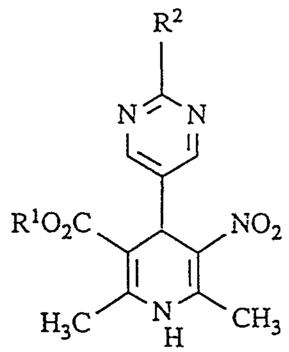

(I)

in welcher

$R^1$ für geradkettiges oder verzweigtes Alkyl mit bis zu 6 C-Atomen steht, das gegebenenfalls substituiert ist durch Phenyl, Cyano, 1 oder mehrere Fluoratome, durch Pyridyl, Thienyl, Furyl oder durch eine Gruppe der Formeln

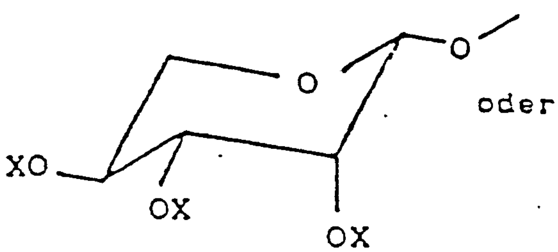

oder

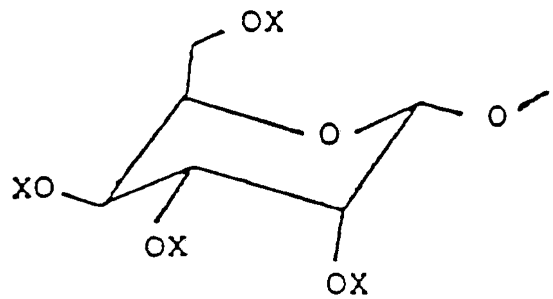

in denen

X Wasserstoff oder Acetyl darstellt,

oder durch eine Aminogruppe der Formel

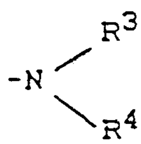

substituiert ist,

wobei

$R^3$, $R^4$ gleich oder verschieden sind und

für Wasserstoff,

für Phenyl,

für Benzyl oder

für $C_1$–$C_3$-Alkyl stehen, und

$R^2$ für den Fall, daß $R^1 \neq$ Methyl, für Wasserstoff steht, oder für geradkettiges oder verzweigtes Alkyl mit bis zu 4 C-Atomen steht, oder für eine Aminogruppe der Formel

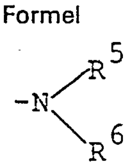

steht, wobei

$R^5$ und $R^6$ gleich oder verschieden sind und Wasserstoff, $C_1$–$C_4$-Alkyl oder $C_2$–$C_4$-Alkenyl darstellen, oder wobei die Substituenten einen Piperazin-, Piperidin-, Morpholin- oder Pyrrolidin-Ring bilden,

sowie deren physiologisch unbedenklichen Salze.

Die erfindungsgemäßen Verbindungen besitzen wertvolle pharmakologische Eigenschaften. Sie sind gefäßdilatierend und positiv inotrop und stellen somit eine Bereicherung der Pharmazie dar.

Physiologisch unbedenkliche Salze können Salze der erfindungsgemäßen Verbindungen mit anorganischen oder organischen Säuren sein. Als Beispiele seien genannt: Salze mit Mineralsäuren wie Halogenwasserstoffsäuren, Schwefelsäure, Phosphorsäure oder mit organischen Säuren wie z.B. Ameisensäure, Essigsäure, Maleinsäure, Fumarsäure, Weinsäure, Äpfelsäure, Zitronensäure, Milchsäure oder Benzoesäure.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie Diastereomerengemische. Die Racemformen lassen sich ebenso wie die Diastereomeren in bekannter Weise in die stereoisomer einheitlichen Bestandteile trennen (vgl. E.L. Eliel, Stereochemisty of Carbon Compounds, McGraw Hill, 1962).

Die erfindungsgemäßen Verbindungen der allgemeinen Formel I, in welcher

$R^1$ und $R^2$ die oben angegebene Bedeutung haben, erhält man, wenn man

(A) Aldehyde der allgemeinen Formel II

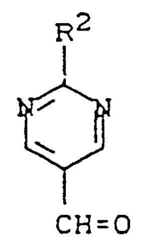

(II),

in welcher

$R^2$ die oben angegebene Bedeutung hat, und Ketoverbindungen der allgemeinen Formel III

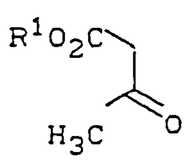

(III),

in welcher

$R^1$ die oben angegebene Bedeutung hat, mit der Verbindung der Formel IV,

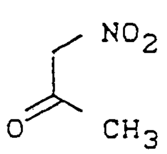

(IV)

und Ammoniak gegebenenfalls in Gegenwart von Wasser oder inerten organischen Lösungsmitteln umsetzt, oder wenn man

(B) Aldehyde der allgemeinen Formel (II) mit Ketoverbindungen der allgemeinen Formel (III) und dem Enamin der Formel (V)

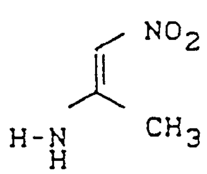

(V),

gegebenenfalls in Gegenwart von Wasser oder inerten organischen Lösungsmitteln umsetzt, oder wenn man

(C) Aldehyde der allgemeinen Formel (II) mit der Ketoverbindung der Formel (IV) und Enaminen der allgemeinen Formel (VI)

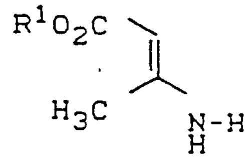

(VI),

in welcher $R^1$ die oben angegebene Bedeutung hat, gegebenenfalls in Gegenwart von Wasser oder inerten organischen Lösungsmitteln umsetzt, oder wenn man

(D) Ketoverbindungen der allgemeinen Formel (III) mit Ammoniak und Ylidenverbindungen der allgemeinen Formel (VII)

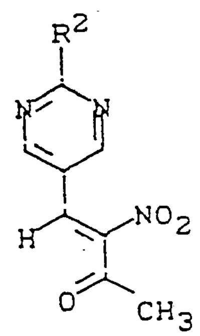

(VII),

in welcher $R^2$ die oben angegebene Bedeutung hat, gegebenenfalls in Gegenwart von Wasser oder inerten organischen Lösungsmitteln umsetzt, oder wenn man

(E) die Ketoverbindung der Formel (IV) mit Ammoniak und Ylidenverbindungen der allgemeinen Formel (VIII)

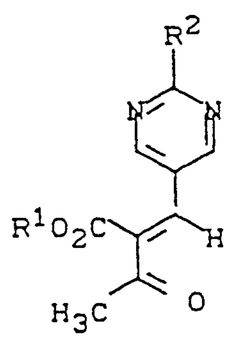

(VIII),

in welcher $R^1$ und $R^2$ die oben angegebene Bedeutung haben, gegebenenfalls in Gegenwart von Wasser oder inerten organischen Lösungsmitteln umsetzt, oder wenn man

(F) Ylidenverbindungen der allgemeinen Formel (VII) mit Enaminen der allgemeinen Formel (VI) gegebenenfalls in Gegenwart von Wasser oder inerten organischen Lösungsmitteln umsetzt, oder wenn man

(G) Ylidenverbindungen der allgemeinen Formel (VIII) mit dem Enamin der Formel (V) gegebenenfalls in Gegenwart von Wasser oder inerten organischen Lösungsmitteln umsetzt.

Je nach Art der verwendeten Ausgangsstoffe können die Synthesen durch folgende Formelschemata wiedergegeben werden:

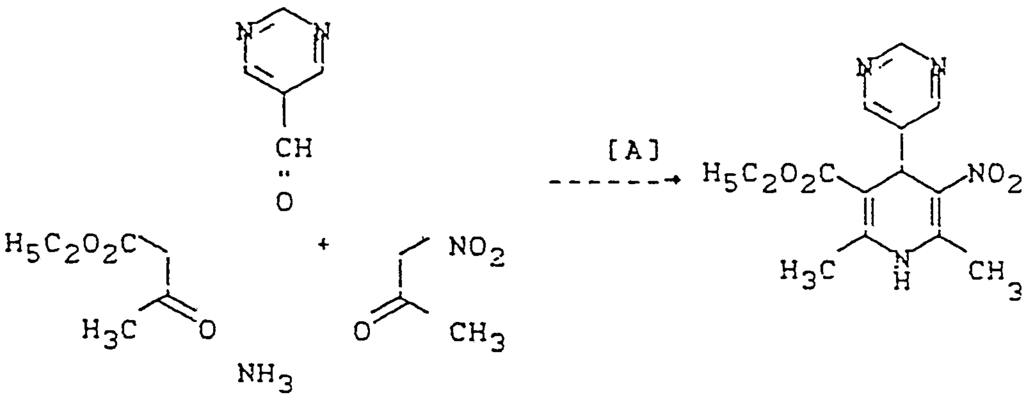
[A]

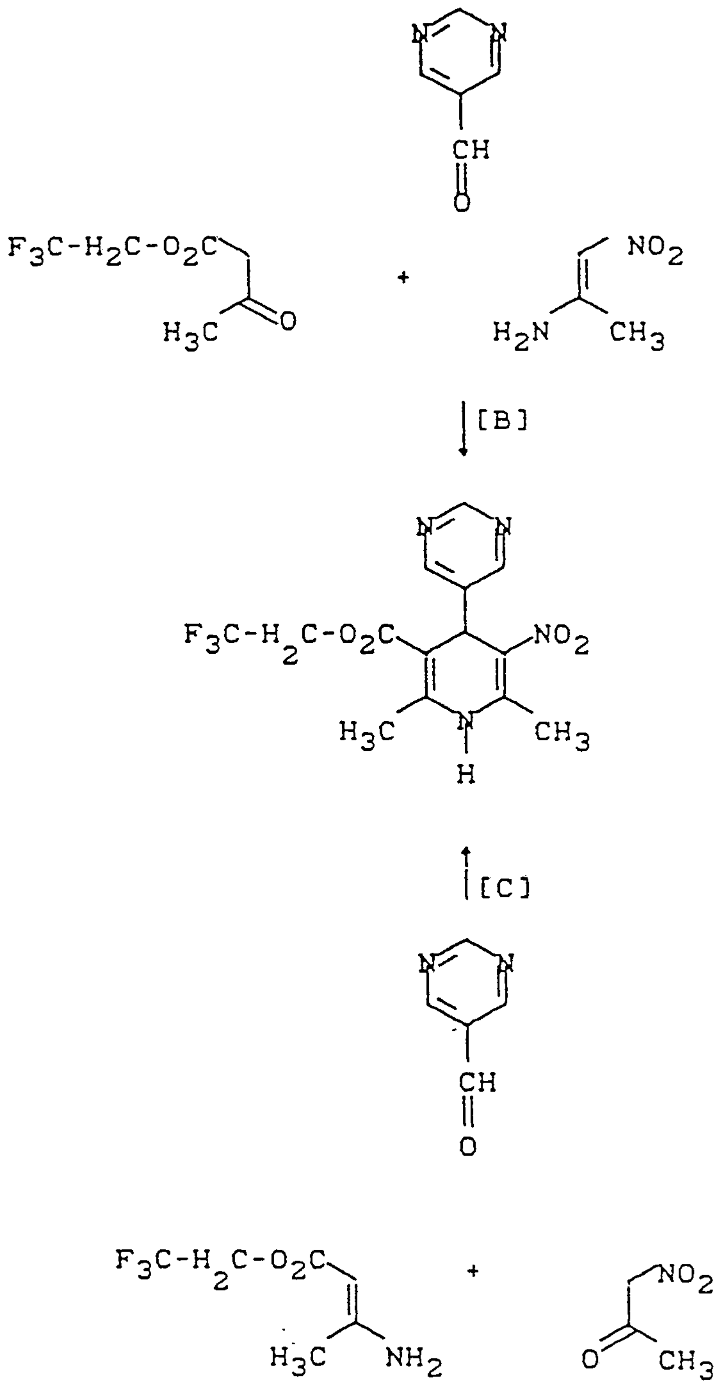
[B]
[C]

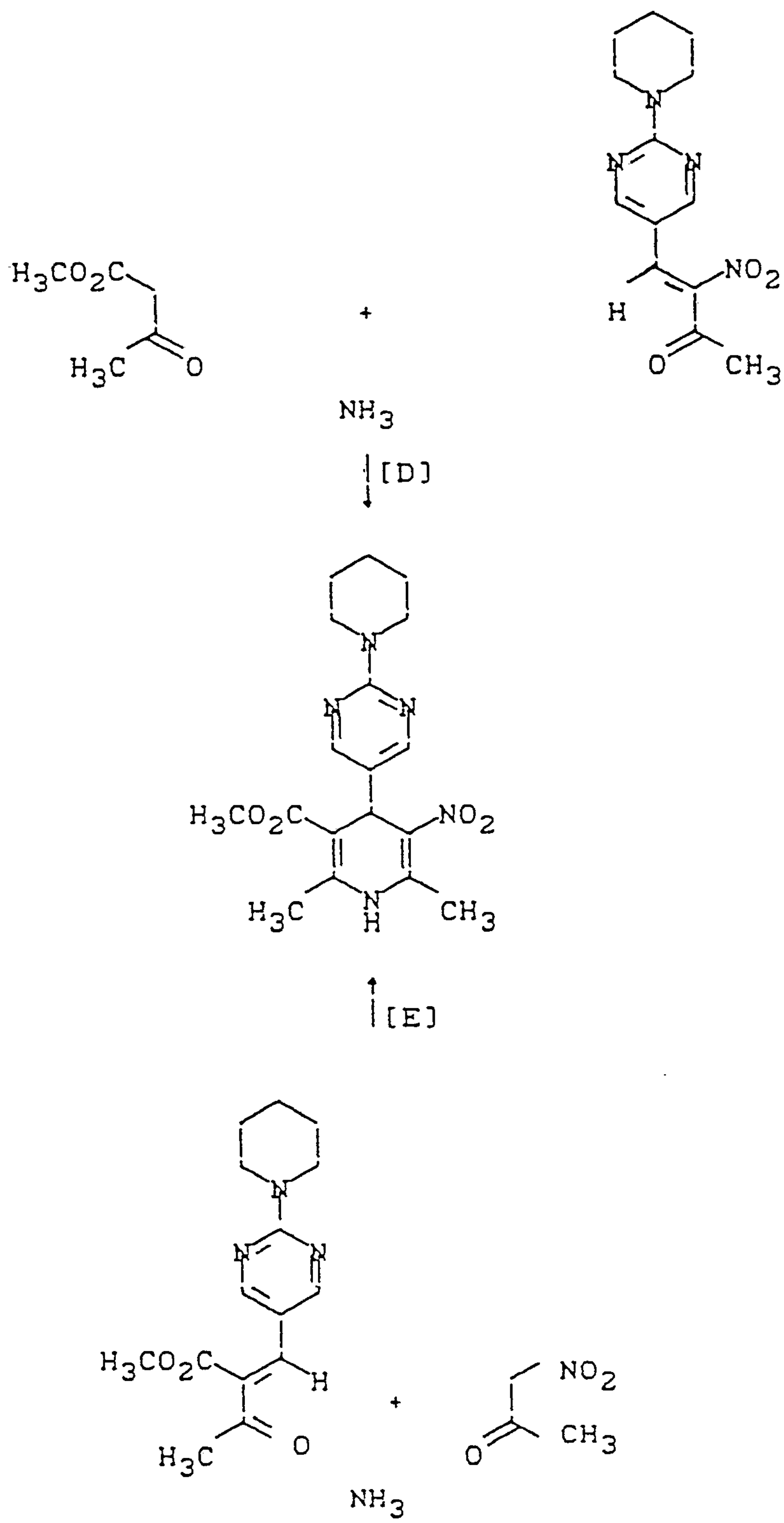
NH3
[D]
[E]
NH3

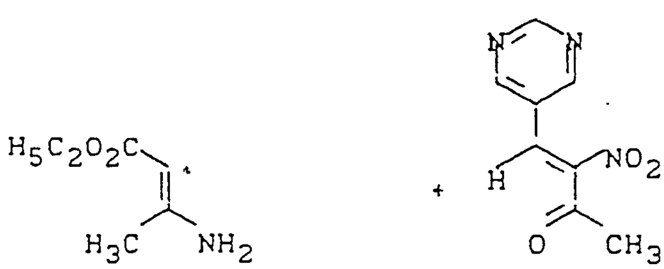

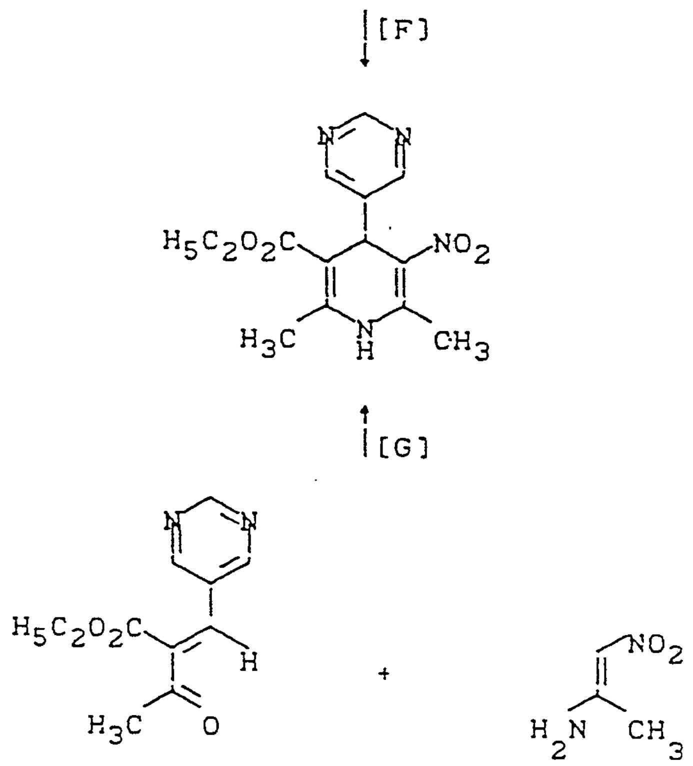


Die Verbindungen der Formeln II–VIII sind bekannt oder können nach literaturbekannten Methoden hergestellt werden. Vergleiche hierzu:

– Aldehyde II: D.J. Brown, The Chemistry of Heterocyclic Compounds, Band «Pyrimidine, Suppl. I», Wiley Interscience, 1970;

– Ketoverbindungen III: D. Borrmann, Houben-Weyl «Methoden der organischen Chemie», Band VII/4, 230 ff. (1968);

– Ketoverbindungen IV: N. Levy, C.W. Scaife, J. Chem. Soc. (London) 1946, 1100: C.D. Hurd, M.E. Nilson, J. Org. Chem. 20, 927 (1955);

– Enamine V: H. Böhme, K.H. Weisel, Arch. Pharm. 310, 30 (1977);

– Enamine VI: S.A. Glickman, A.C. Cope, J. Am. Chem. Soc. 67, 1017 (1945);

– Ylidenverbindungen VII: A. Dornow, W. Sassenberg, Liebigs Ann. Chem. 602, 14 (1957);

– Ylidenverbindungen VIII: Organic Reactions XV, 204 (1967).

Als Verdünnungsmittel kommen für alle Verfahren Wasser oder alle inerten organischen Lösungsmittel infrage. Hierzu gehören bevorzugt Alkohole wie Methanol, Ethanol, Propanol, oder Isopropanol, Ether wie Diethylether, Dioxan, Tetrahydrofuran oder Glykolmonomethylether, Eisessig, Pyridin, Dimethylformamid, Acetonitril, Dimethylsulfoxid oder Hexamethylphosphorsäuretriamid.

Die Reaktionstemperaturen für alle Verfahren können in einem grösseren Bereich variiert werden. Im allgemeinen arbeitet man in einem Bereich von 10 bis 200 °C, bevorzugt von 20 bis 150 °C.

Die Umsetzung kann bei Normaldruck, aber auch bei erhöhtem Druck durchgeführt werden. Im allgemeinen arbeitet man bei Normaldruck.

Bei der Durchführung der erfindungsgemäßen Verfahren ist das Verhältnis der an der Reaktion beteiligten Stoffe beliebig. Im allgemeinen arbeitet man jedoch mit molaren Mengen der Reaktanden.

Die vorstehenden Herstellungsverfahren sind lediglich zur Verdeutlichung angegeben. Die Herstellung der Verbindungen der Formel I ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen haben eine positiv inotrope Wirkung und zeigen somit ein nicht vorhersehbares und wertvolles pharmakologisches Wirkspektrum. Die können als kreislaufbeeinflussende Mittel, als Koronartherapeutika, Antiarrhythmika und Antihypotonika sowie zur Behandlung von Herzinsuffizienz eingesetzt werden.

Die Herz- und Gefäßwirkungen wurden am isoliert perfundierten Herzen des Meerschweinchens gefunden.

Dazu werden die Herzen von 250 bis 350 g schweren Albino-Meerschweinchen verwendet. Die Tiere werden mit einem Schlag auf den Kopf getötet, der Thorax geöffnet, in die freipräparierte Aorta eine Metallkanüle eingebunden und der linke Vorhof eröffnet. Das Herz wird mit den Lungen aus dem Thorax herausgetrennt und über die Aortenkanüle an die Perfusionsapparatur bei laufender Perfusion angeschlossen. Die Lungen werden an den Lungenwurzeln abgetrennt. Als Perfusionsmedium dient Krebs-Henseleit-Lösung (1) (118,5 mmol/l NaCl, 4,75 mmol/l KCl, 1,19 (mmol/l) $KH_2PO_4$, 119 mmol/l $MgSO_4$, 25 mmol/l $NaHCO_3$,

0,013 mmol/l NaEDTA), deren $CaCl_2$ je nach Bedarf variiert wird, in der Regel jedoch 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% $O_2$, 5% $CO_2$ zur Aufrechterhaltung des pH-Wertes von 7,4) begast. Die Herzen werden mit konstantem Fluß (10 ml/min) bei 32 °C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt und die isovolumetrischen Kontraktionen auf einem Schnellschreiber registriert [Opie, L., J. Physiol. 180 (1965) 529–541]. Der Perfusionsdruck wird mittels eines Druckaufnehmers, der vor dem Herzen mit dem Perfusionssystem in Verbindung steht, registriert. Unter diesen Bedingungen zeigt eine Senkung des Perfusionsdruckes eine Koronardilatation, eine Steigerung der linksventrikulären Druckamplitude einen Anstieg der Herzkontraktilität ein. Die erfindungsgemäßen Verbindungen werden in geeigneten Verdünnungen in das Perfusionssystem kurz vor dem isolierten Herzen infudiert.

Die folgenden Werte zeigen beispielhaft den kontraktilitätssteigernden Effekt der erfindungsgemäßen Verbindungen am isoliert perfundierten Meerschweinchenherzen:

| Bsp.-Nr. | Konzentration (g/ml) | prozentuale Änderung der Kontraktionsamplitude |
|---|---|---|
| 1 | $10^{-7}$ | + 5 |
| 1 | $10^{-6}$ | +22 |
| 1 | $10^{-5}$ | +89 |

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether (z.B. Lignin, Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendung gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen. Bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Herstellungsbeispiele

Allgemeine Arbeitsvorschrift A:

10 mmol Pyrimidin-5-carbaldehyd, 10 mmol β-Aminocrotonsäureester und 20 mmol Nitroaceton werden in 20 ml i-Propanol 4 h auf 60 °C erhitzt. Nach Abdampfen des Lösungsmittels wird der Rückstand an Kieselgel mit $CHCl_3$ mit 5% Methanol chromatographiert. Die eingedampften

Produktfraktionen werden aus i-Propanol/Petrolether kristallisiert.

Allgemeine Arbeitsvorschrift B:

10 mmol 2-Nitro-1-(pyrimidin-5-yl)buten-1-on-3 und 10 mmol β-Aminocrotonsäureester werden in 15 ml i-Propanol 3 h auf 60 °C erhitzt. Teilweise kristallisieren die Endprodukte direkt aus. Andernfalls erfolgt chromatographische Aufarbeitung wie nach Arbeitsvorschrift A.

Allgemeine Arbeitsvorschrift C:

10 mmol α-Acetyl-β-(pyrimidin-5-yl)acrylsäureester und 15 mmol $NH_3$-Nitroaceton-Komplex (entspricht 2-Amino-1-nitropropen-1) werden in 15 ml i-Propanol 3 h bei 60 °C erhitzt. Teilweise kristallisieren die Endprodukte direkt aus. Andernfalls erfolgt chromatographische Aufarbeitung wie nach Arbeitsvorschrift A.

Die Herstellung von 2-Nitro-1-(pyrimidin-5-yl)-buten-1-on-3-Derivaten (Vorschrift B) sowie von α-Acetyl-β-(pyrimidin-5-yl)acrylsäureestern (Vorschrift C) erfolgt nach üblichen Methoden durch direkte Kondensation von Acetessigestern mit Pyrimidin-5-carbaldehyden unter saurer Katalyse.

Die in der nachfolgenden Tabelle aufgeführten Verbindungen wurden nach allen vorstehend beschriebenen Verfahren hergestellt.

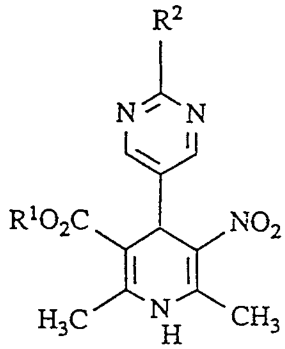

(I)

| Bsp.-Nr. | $R^1$ | $R^2$ | Schmelzpunkt (°C) |
|---|---|---|---|
| 1 | $-CH_2-CH_2-$ 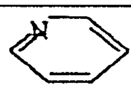 | H | 190 |
| 2 | $-CH(CH_3)_2$ | H | 240 |
| 3 | $-(CH_2)_3-CH_3$ | H | 210 |
| 4 | $-CH_2-CH_3$ | H | 250 |
| 5 | $-CH_2-CH_2-CN$ | H | 222 |
| 6 | $-CH_2-CF_3$ | H | 227 |
| 7 | $-(CH_2)_2-N(CH_3)CH_2-$ 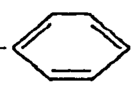 | H | 222 |
| 8 | $-CH_3$ | $-NH-CH_2-CH=CH_2$ | 226 |
| 9 | $-CH_3$ | 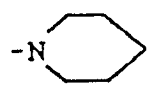 | 244 |
| 10 | 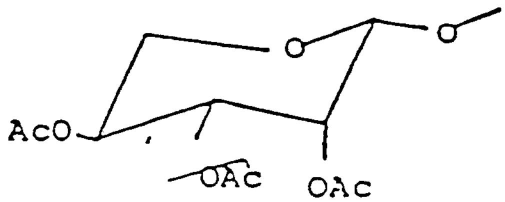 $(CH_2)_3-$ | H | Harz |
| 11 | 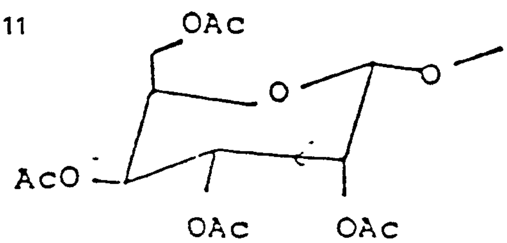 $(CH_2)_2-$ | H | Harz |

**Patentansprüche**

1. 4-Pyrimidinyl-1,4-dihydropyridine der allgemeinen Formel I

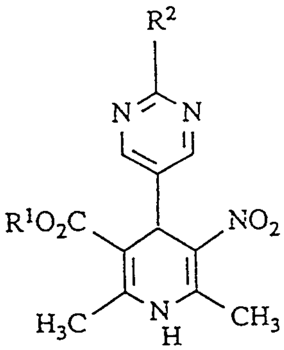

(I)

in welcher

$R^1$ für geradkettiges oder verzweigtes Alkyl mit bis zu 6 C-Atomen steht, das gegebenenfalls substituiert ist durch Phenyl, Cyano, 1 oder mehrere Fluoratome, durch Pyridyl, Thienyl, Furyl oder durch eine Gruppe der Formeln

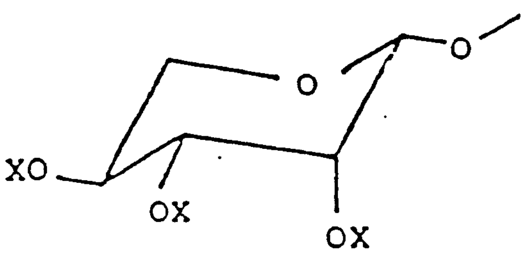

oder

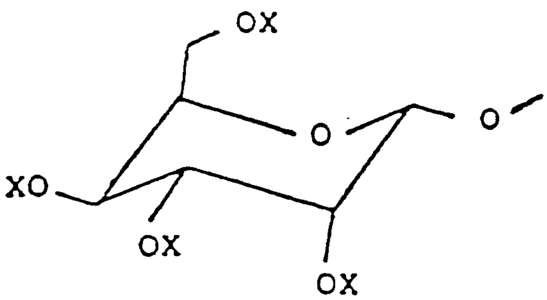

in denen

X Wasserstoff oder Acetyl darstellt,

oder durch eine Aminogruppe der Formel

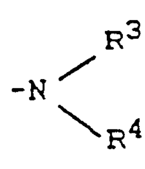

substituiert ist, wobei

$R^3$, $R^4$ gleich oder verschieden sind und

für Wasserstoff,

für Phenyl,

für Benzyl oder

für $C_1$–$C_3$-Alkyl stehen, und

$R^2$ für den Fall, daß $R^1 \neq$ Methyl, für Wasserstoff steht, oder für geradkettiges oder verzweigtes Alkyl mit bis zu 4 C-Atomen steht, oder für eine Aminogruppe der Formel

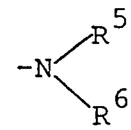

steht, wobei

$R^5$ und $R^6$ gleich oder verschieden sind und Wasserstoff, $C_1$–$C_4$-Alkyl oder $C_2$–$C_4$-Alkenyl darstellen, oder wobei die Substituenten einen Piperazin-, Piperidin-, Morpholin- oder Pyrrolidin-Ring bilden,

sowie deren physiologisch unbedenklichen Salze.

2. Verbindungen gemäß Anspruch 1 zur Bekämpfung von Herz- und Kreislauferkrankungen.

3. Verfahren zur Herstellung von 4-Pyrimidinyl-1,4-dihydropyridine der allgemeinen Formel I

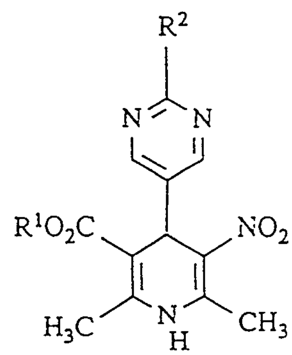

(I)

in welcher

$R^1$ für geradkettiges oder verzweigtes Alkyl mit bis zu 6 C-Atomen steht, das gegebenenfalls substituiert ist durch Phenyl, Cyano, 1 oder mehrere Fluoratome, durch Pyridyl, Thienyl, Furyl oder durch eine Gruppe der Formeln

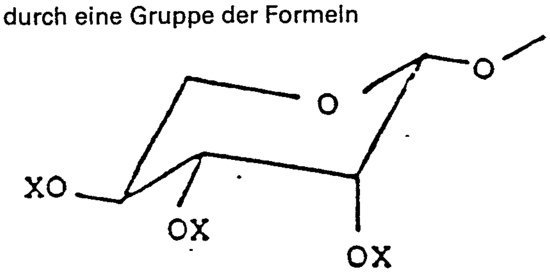

oder

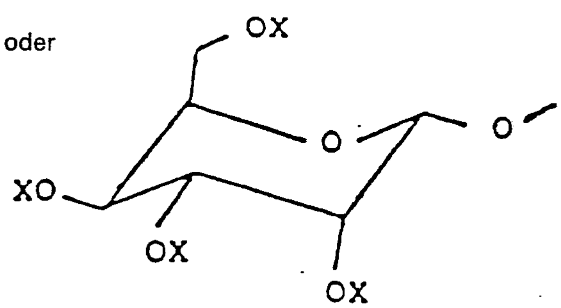

in denen

X Wasserstoff oder Acetyl darstellt,

oder durch eine Aminogruppe der Formel

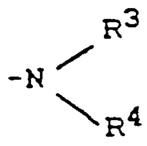

substituiert ist, wobei

$R^3$, $R^4$ gleich oder verschieden sind und
für Wasserstoff,
für Phenyl,
für Benzyl oder
für $C_1$–$C_3$-Alkyl stehen, und

$R^2$ für den Fall, daß $R^1 \neq$ Methyl, für Wasserstoff steht, oder für geradkettiges oder verzweigtes Alkyl mit bis zu 4 C-Atomen steht, oder für eine Aminogruppe der Formel

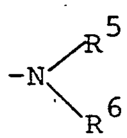

steht, wobei

$R^5$ und $R^6$ gleich oder verschieden sind und Wasserstoff, $C_1$–$C_4$-Alkyl oder $C_2$–$C_4$-Alkenyl darstellen, oder wobei die Substituenten einen Piperazin-, Piperidin-, Morpholin- oder Pyrrolidin-Ring bilden,

sowie deren physiologisch unbedenklichen Salze, dadurch gekennzeichnet, daß man

(A) Aldehyde der allgemeinen Formel II

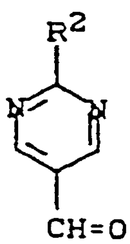

(II),

in welcher

$R^2$ die oben angegebene Bedeutung hat, und Ketoverbindungen der allgemeinen Formel III

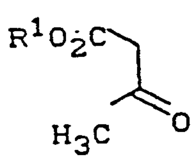

(III),

in welcher

$R^1$ die oben angegebene Bedeutung hat, mit der Verbindung der Formel IV

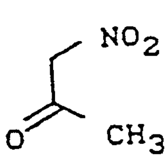

(IV)

und Ammoniak

gegebenenfalls in Gegenwart von Wasser oder inerter organischer Lösungsmittel umsetzt, oder daß man

(B) Aldehyde der allgemeinen Formel (II) mit Ketoverbindungen der allgemeinen Formel (III) und dem Enamin der Formel (V)

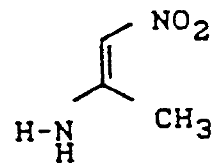

(V),

gegebenenfalls in Gegenwart von Wasser oder inerter organischer Lösungsmittel umsetzt, oder daß man

(C) Aldehyde der allgemeinen Formel (II) mit der Ketoverbindung der Formel (IV) und Enaminen der allgemeinen Formel (VI)

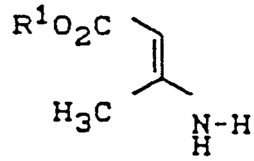

(VI),

in welcher $R^1$ die oben angegebene Bedeutung hat, gegebenenfalls in Gegenwart von Wasser oder inerter organischer Lösungsmittel umsetzt, oder daß man

(D) Ketoverbindungen der allgemeinen Formel (III) mit Ammoniak und Ylidenverbindungen der allgemeinen Formel (VII)

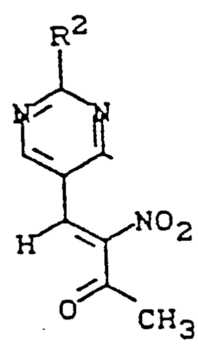

(VII),

in welcher $R^2$ die oben angegebene Bedeutung hat, gegebenenfalls in Gegenwart von Wasser oder inerter organischer Lösungsmittel umsetzt, oder daß man

(E) die Ketoverbindung der Formel (IV) mit Ammoniak und Ylidenverbindungen der allgemeinen Formel (VIII)

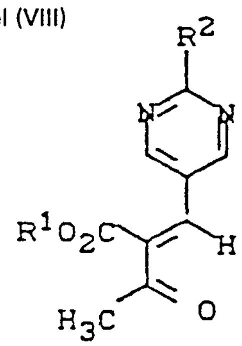

(VIII),

in welcher $R^1$ und $R^2$ die oben angegebene Bedeutung haben, gegebenenfalls in Gegenwart von Wasser oder inerter organischer Lösungsmittel umsetzt, oder daß man

(F) Ylidenverbindungen der allgemeinen Formel (VII) mit Enaminen der allgemeinen Formel (VI) gegebenenfalls in Gegenwart von Wasser oder inerter organischer Lösungsmittel umsetzt, oder daß man

(G) Ylidenverbindungen der allgemeinen Formel (VIII) mit dem Enamin der Formel (V) gegebenenfalls in Gegenwart von Wasser oder inerter organischer Lösungsmittel umsetzt.

4. Verfahren gemäß Anspruch 3, dadurch gekennzeichnet, daß die Reaktion bei Temperaturen zwischen 10 und 200 °C durchgeführt wird.

5. Arzneimittel enthaltend mindestens eine Verbindung gemäß Anspruch 1.

6. Verfahren zur Herstellung von Arzneimitteln, dadurch gekennzeichnet, daß man mindestens eine Verbindung gemäß Anspruch 1 gegebenenfalls unter Verwendung von üblichen Hilfs- und Trägerstoffen in eine geeignete Applikationsform überführt.

7. Verwendung von Verbindungen gemäß Anspruch 1 bei der Herstellung von herz- und kreislaufwirksamen Arzneimitteln.

**Claims**

1. 4-Pyrimidinyl-1,4-dihydropyridines of the general formula I

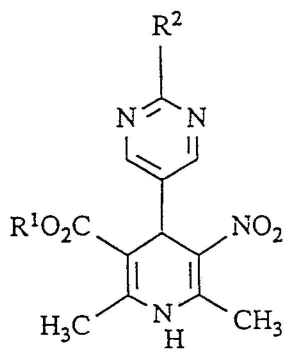

(I),

in which

$R^1$ represents straight-chain or branched alkyl which has up to 6 C atomes and is optionally substituted by phenyl, cyano, 1 or more fluorine atoms, by pyridyl, thienyl, furyl, or by a group of the formulae

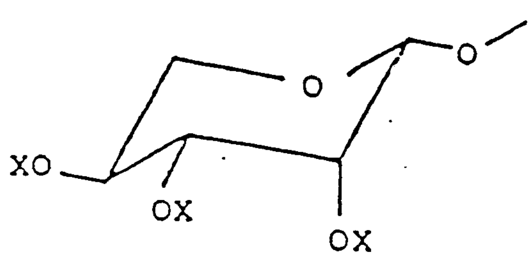

or

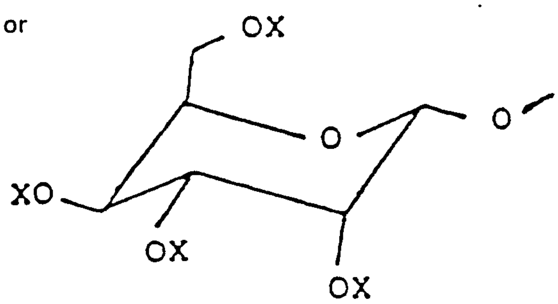

in which

X represents hydrogen or acetyl,

or is substituted by an amino group of the formula

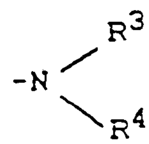

wherein

$R^3$ and $R^4$ are identical or different and represent hydrogen, or represent phenyl, or represent benzyl, or represent $C_1$–$C_3$-alkyl, and

$R^2$ in the case where $R^1 \neq$ methyl, represents hydrogen, or represents straight-chain or branched alkyl which has up to 4 C atoms, or represents an amino group of the formula

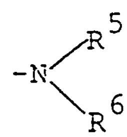

wherein

$R^5$ and $R^6$ are identical or different and represent hydrogen, $C_1$–$C_4$-alkyl or $C_2$–$C_4$-alkenyl,

or wherein

the substituents form a piperazine, piperidine, morpholine or pyrrolidine ring,

and physiologically acceptable salts thereof.

2. Compounds according to claim 1 for combating cardiac and circulatory diseases.

3. Process for the preparation of 4-pyrimidinyl-1,4-dihydropyridines of the general formula I

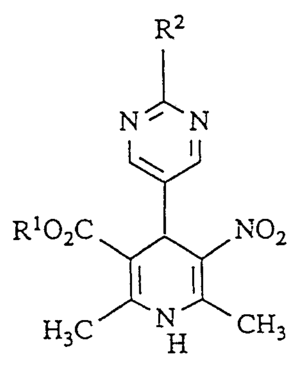

(I),

in which

$R_1$ represents straight-chain or branched alkyl which has up to 6 C atoms and is optionally

substituted by phenyl, cyano, 1 or more fluorine atoms, by pyridyl, thienyl, furyl, or by a group of the formulae

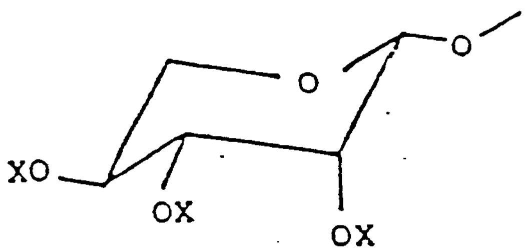

or

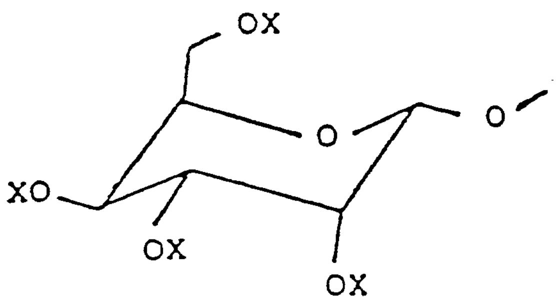

in which

X represents hydrogen or acetyl,

or is substituted by an amino group of the formula

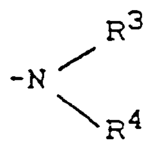

wherein

$R^3$ and $R^4$ are identical or different and represent hydrogen, or represent phenyl, or represent benzyl, or represent $C_3$–$C_4$-alkyl, and

$R^2$ in the case where $R^1 \neq$ methyl, represents hydrogen, or represents straight-chain or branched alkyl which has up to 4 C atoms, or represents an amino group of the formula

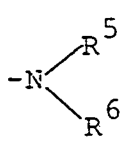

wherein

$R^5$ and $R^6$ are identical or different and represent hydrogen, $C_1$–$C_4$-alkyl or $C_2$–$C_4$-alkenyl,

or wherein

the substituents form a piperazine, piperidine, morpholine or pyrrolidine ring,

and physiologically acceptable salts thereof, characterised in that

(A) aldehydes of the general formula II

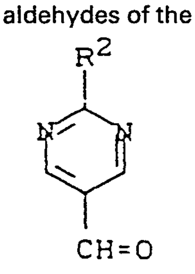

(II),

in which

$R^2$ has the abovementioned meaning, and keto compounds of the general formula III

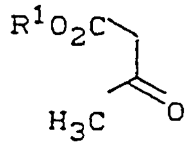

(III),

in which

$R^1$ has the abovementioned meaning, are reacted with the compound of the formula IV

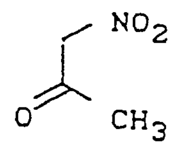

(IV)

and ammonia, if appropriate in the presence of water or inert organic solvents, or in that

(B) aldehydes of the general formula (II) are reacted with keto compounds of the general formula (III) and the enamine of the formula (V)

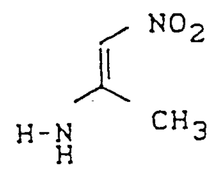

(V),

if appropriate in the presence of water or inert organic solvents, or in that

(C) aldehydes of the general formula (II) are reacted with the keto compound of the formula (IV) and enamines of the general formula (VI)

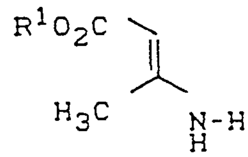

(VI),

in which

$R^1$ has the abovementioned meaning,

if appropriate in the presence of water or inert organic solvents, or in that

(D) keto compounds of the general formula (III) are reacted with ammonia and ylidene compounds of the general formula (VII)

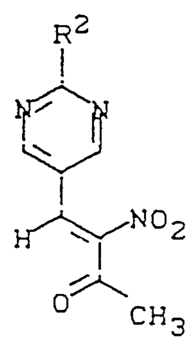

(VII),

in which

$R^2$ has the abovementioned meaning,

if appropriate in the presence of water or inert organic solvents, or in that

(E) the keto compound of the formula (IV) is reacted with ammonia and ylidene compounds of the general formula (VIII)

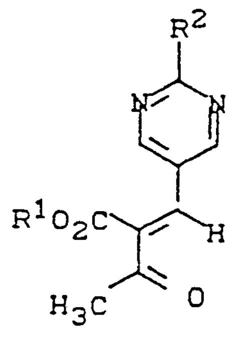

(VIII),

in which

$R^1$ and $R^2$ have the abovementioned meaning,

if appropriate in the presence of water or inert organic solvents, or in that

(F) ylidene compounds of the general formula (VII) are reacted with enamines of the general formula (VI), if appropriate in the presence of water or inert organic solvents, or in that

(G) ylidene compounds of the general formula (VIII) are reacted with the enamine of the formula (V), if appropriate in the presence of water or inert organic solvents.

4. Process according to claim 3, characterised in that the reaction is carried out at temperatures of between 10 and 200 °C.

5. Medicaments containing at least one compound according to claim 1.

6. Process for the preparation of medicaments, characterised in that at least one compound according to claim 1 is converted into a suitable administration form, if appropriate with the use of customary auxiliaries and/or excipients.

7. Use of 7 compounds according to claim 1 in the preparation of medicaments which have an effect on the heart and/or circulation.

**Revendications**

1. 4-pyrimidinyl-1,4-dihydropyridine de formule générale I

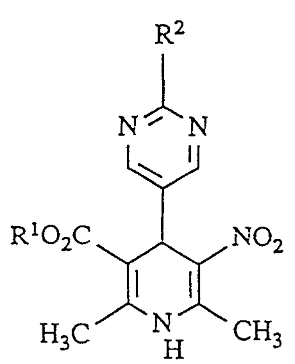

(I),

dans laquelle

$R^1$ est un groupe alkyle à chaîne droite ou à chaîne ramifiée ayant jusqu'à 6 atomes de carbone, qui est éventuellement substitué par un groupe phényle, cyano, un ou plusieurs atomes de fluor, un groupe pyridyle, thiényle, furyle ou par un groupe de formules

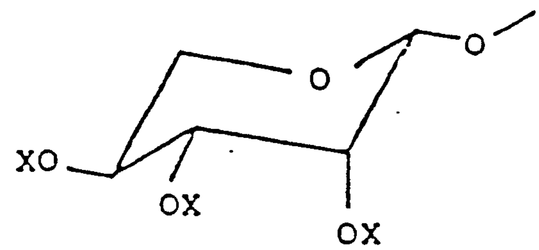

ou

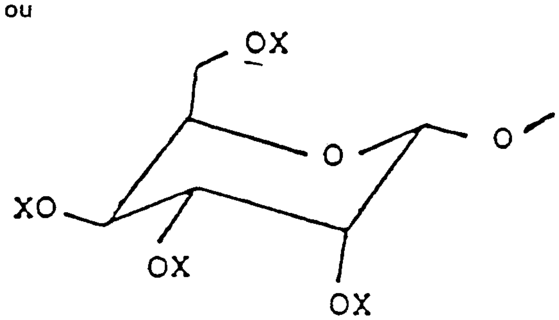

dans lesquelles

X est l'hydrogène ou le groupe acétyle, ou par un groupe amine de formule

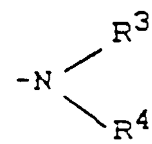

dans laquelle

$R^3$, $R^4$ sont identiques ou différents et représentent

l'hydrogène,

le groupe phényle,

le groupe benzyle ou

un groupe alkyle en $C_1$ à $CV_3$, et

$R^2$ au cas où $R^1$ n'est pas un groupe méthyle, représente l'hydrogène ou un groupe alkyle à chaîne droite ou à chaîne ramifiée ayant jusqu'à 4 atomes de carbone, ou un groupe amino de formule

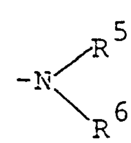

dans laquelle

$R^5$ et $R^6$ sont identiques ou différents et représentent l'hydrogène, un groupe alkyle en $C_1$ à $C_4$ ou un groupe alcényle en $C_2$ à $C_4$, ou bien les substituants forment un noyau de pipérazine, de pipéridine, de morpholine ou de pyrrolidine,

ainsi que leurs sels acceptables du point de vue physiologique.

2. Composés suivant la revendication 1, destinés à combattre des troubles cardiaques et circulatoires.

3. Procédé de préparation de 4-pyrimidinyl-1,4-dihydropyridines de formule générale I

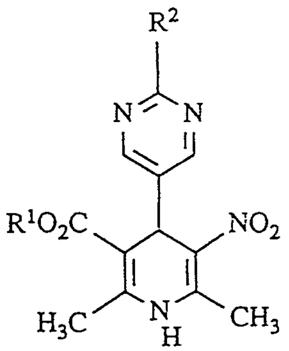

(I),

dans laquelle

$R^1$ est un groupe alkyle à chaîne droite ou à chaîne ramifiée ayant jusqu'à 6 atomes de carbone, qui est éventuellement substitué par un groupe phényle, cyano, un ou plusieurs atomes de fluor, un groupe pyridyle, thiényle, furyle ou par un groupe de formules

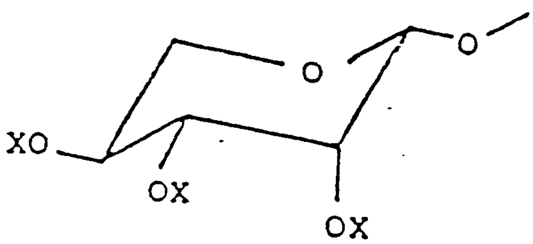

ou

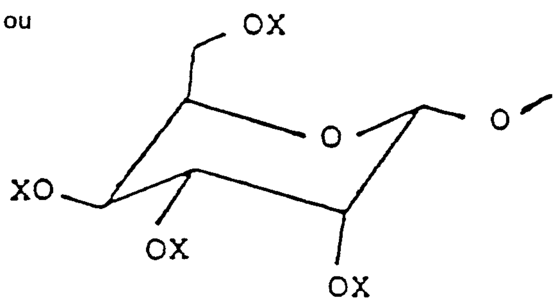

dans lesquelles

X est l'hydrogène ou le groupe acétyle, ou par un groupe amino de formule

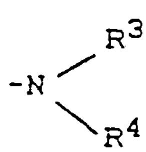

dans laquelle

$R^3$, $R^4$ sont identiques ou différents et représentent

l'hydrogène

le groupe phényle,

le groupe benzyle ou

un groupe alkyle en $C_1$ à $C_3$, et

$R^2$ au cas où $R^1$ n'est pas un groupe méthyle, représente l'hydrogène ou un groupe alkyle à chaîne droite ou à chaîne ramifiée ayant jusqu'à 4 atomes de carbone, ou un groupe amino de formule

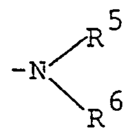

dans laquelle

$R^5$ et $R^6$ sont identiques ou différents et représentent l'hydrogène, un groupe alkyle en $C_1$ à $C_4$ ou un groupe alcényle en $C_2$ à $C_4$, ou bien les substituants forment un noyau de pipérazine, de pipéridine, de morpholine ou de pyrrolidine,

ainsi que leurs sels acceptables du point de vue physiologique, caractérisé en ce que

(A) on fait réagir des aldéhydes de formule générale II

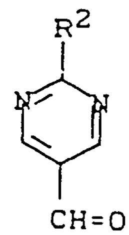

(II),

dans laquelle

$R^2$ a la définition indiquée ci-dessus, et des composés cétoniques de formule générale III

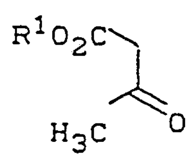

(III),

dans laquelle

$R^1$ a la définition indiquée ci-dessus, avec le composé de formule IV

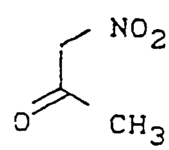

(IV)

et l'ammoniac

le cas échéant en présence d'eau et de solvants organiques inertes, ou bien

(B) on fait réagir des aldéhydes de formule générale (II) avec des composés cétoniques de formule générale (III) et l'énamine de formule (V)

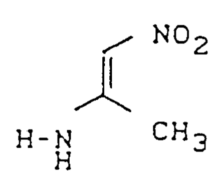

(V),

le cas échéant en présence d'eau ou de solvants organiques inertes, ou bien

(C) on fait réagir des aldéhydes de formule générale (II) avec le composé cétonique de formule (IV) et des énamines de formule générale (VI)

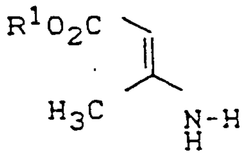

(VI),

dans laquelle $R^1$ a la définition indiquée ci-dessus, éventuellement en présence d'eau ou de solvants organiques inertes, ou bien

(D) on fait réagir des composés cétoniques de formule générale (III) avec l'ammoniac et des composés ylidéniques de formule générale (VII)

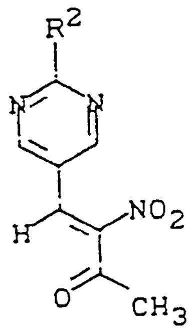

(VII),

dans laquelle $R^2$ a la définition indiquée ci-dessus, éventuellement en présence d'eau ou de solvants organiques inertes, ou bien

(E) on fait réagir le composé cétonique de formule (IV) avec l'ammoniac et des composés ylidéniques de formule générale (VIII)

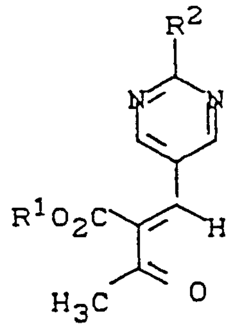

(VIII),

dans laquelle $R^1$ et $R^2$ ont la définition indiquée ci-dessus, le cas échéant en présence d'eau ou de solvants organiques inertes, ou bien

(F) on fait réagir des composés ylidéniques de formule générale (VII) avec des énamines de formule générale (VI), éventuellement en présence d'eau ou de solvants organiques inertes, ou bien

(G) on fait réagir des composés ylidéniques de formule générale (VIII) avec l'énamine de formule (V), éventuellement en présence d'eau ou de solvants organiques inertes.

4. Procédé suivant la revendication 3, caractérisé en ce que la réaction est conduite à des températures comprises entre 10 et 200 °C.

5. Médicament contenant au moins un composé suivant la revendication 1.

6. Procédé de préparation de médicaments, caractérisé en ce qu'on incorpore dans une forme d'administration appropriée au moins un composé suivant la revendication 1, en utilisant éventuellement des substances auxiliaires et des supports classiques.

7. Utilisation de composés suivant la revendication 1 dans la préparation de médicaments agissant sur le cœur et sur le système circulatoire.